# EUROPÄISCHE PATENTSCHRIFT

(11) **EP 0 930 509 B1**
(45) Veröffentlichungstag und Bekanntmachung des Hinweises auf die Patenterteilung: **17.03.2004**
(21) Anmeldenummer: 98204162.6
(22) Anmeldetag: 08.12.1998
(51) Int. Cl.: G01R 33/28, A61B 5/055

(54) **MR-Anordnung mit einem medizinischen Instrument und Verfahren zur Positionsbestimmung des medizinischen Instruments**
MR device comprising a medical instrument and method for determining the location of the medical instrument
Appareil de résonance magnétique avec un instrument médical et procédé pour déterminer la position de l'instrument médical

(30) Priorität: 16.12.1997 DE 19755782
(43) Veröffentlichungstag der Anmeldung: 21.07.1999
(73) Patentinhaber: Philips Intellectual Property & Standards GmbH, 20099 Hamburg (DE); Koninklijke Philips Electronics N.V., 5621 BA Eindhoven (NL)
(72) Erfinder: Lüdeke, Kai-Michael, Philips Patentverwaltung GmbH, 22335 Hamburg (DE); Rasche, Volker Dr., Philips Patentverwaltung GmbH, 22335 Hamburg (DE)
(74) Vertreter: Peters, Carl Heinrich, Dipl.-Ing.

(56) Entgegenhaltungen:
- EP-A- 0 425 319
- EP-A- 0 731 362
- EP-A- 0 768 539
- US-A- 5 318 025
- GRANT D.M, HARRIS, R.K, ED.: "Encyclopedia of Magnetic Resonance" 1996 , JOHN WILEY & SONS , CHICHESTER, GB XP002158420 * vol. 2, p. 1373-1378, Hurst, G.C., Misic, G.J.: "Coils for Insertion into the Human Body" *

## Beschreibung

Die Erfindung betrifft eine Magnetresonanz (MR)-Anordnung gemäß dem Oberbegriff des Anspruchs 1. Die Erfindung betrifft außerdem ein medizinisches Instrument zum Einführen in ein Untersuchungsobjekt, sowie ein Verfahren zur Positionsbestimmung eines in ein Untersuchungsobjekt einführbaren medizinischen Instruments.

Eine ähnliche MR-Anordnung, medizinisches Instrument und Verfahren zu dessen Positionsbestimmung sind aus der US 5,353,795 bekannt. Dort ist eine kleine Hochfrequenzspule, eine sogenannte Mikrospule, in einem Katheter angeordnet, der in einen Patienten eingeführt wird. In die Mikrospule wird bei Betrieb der MR-Anordnung nach Anregung des Untersuchungsbereich ein hochfrequentes Signal induziert, das mittels einer Hochfrequenzleitung einer Empfängeranordnung zugeführt wird, die das Signal weiterarbeitet und die Position der Spule ermittelt. Diese Position kann dann in ein Bild, beispielsweise ein MR-Bild oder ein Computertomographie (CT)-Bild, eingeblendet werden.

Bei der bekannten Anordnung erweist sich die erforderliche Hochfrequenzleitung von der außerhalb des Untersuchungsobjekts angeordneten Sender- und/oder Empfangsanordnung zu einer bevorzugt an der Spitze des medizinischen Instruments angeordneten Mikrospule als nachteilig. Es besteht die Gefahr von Gewebeaufheizungen durch entstehende Resonanzen (insbesondere λ/4-Resonanzen) in der Umgebung der Hochfrequenzleitung während der Sendephase (HF-Anregung) der MR-Untersuchung. Die Hochfrequenzleitung muß weiterhin zwangsweise durch sehr dünne Drähte gebildet werden, da das medizinische Instrument je nach Anwendung auch in sehr dünne Adern eingeführt werden muß. Dadurch können größere Signalverluste bei der Übertragung des empfangenen Signals von der Mikrospule zu einer Empfangsanordnung auftreten. Die Mikrospule muß zusammen mit der Hochfrequenzleitung außerdem einen stabilen Resonanzkreis bilden, weshalb die Länge der Hochfrequenzleitung nicht beliebig verändert werden kann. Bei der bekannten Anordnung wird außerdem ein separater Empfangskanal für die Mikrospule oder (bei Nutzung eines einzigen Empfangskanals) eine Umschaltvorrichtung zum Umschalten zwischen der Empfangsspulenanordnung und der Mikrospule benötigt.

Weiterhin ist aus der EP-A-0 768 539 eine MR-Anordnung gemäß dem Oberbegriff des Anspruchs 1 bekannt, bei der die in der Umgebung einer an einem in den Körper einführbaren Instrument befestigten Mikrospule (im Vergleich zu den anderen Bereichen) erhöhte Kernmagnetisierung zur Positionsbestimmung des Instruments ausgenutzt wird. Diese MR-Anordnung benötigt zwar keine Hochfrequenzleitungen zum Anschluß der Mikrospule; die Positionsbestimmung kann dabei aber nicht unmittelbar anhand eines von der Mikrospule gelieferten Signals erfolgen, sondern ergibt sich aus einem MR-Bild, das aus den aus dem Körper des Patienten empfangenen MR-Signalen rekonstruiert wird.

Der vorliegenden Erfindung liegt die Aufgabe zugrunde, eine verbesserte MR-Anordnung, ein verbessertes medizinisches Instrument und ein verbessertes Verfahren zur Positionsbestimmung eines solchen medizinischen Instruments anzugeben, wobei insbesondere die genannten Nachteile vermieden werden sollen.

Diese Aufgabe wird hinsichtlich der MR-Anordnung und hinsichtlich des medizinischen Instruments erfindungsgemäß durch die Maßnahmen nach Anspruch 1 bzw. 10 gelöst.

Die Hochfrequenzleitung zu einer außerhalb des Untersuchungsobjekts angeordneten Senderanordnung sowie die Senderanordnung selbst können bei der erfindungsgemäßen MR-Anordnung entfallen, da erfindungsgemäß das in die Spulenanordnung induzierte Signal nach einer Hochfrequenz-Anregung des im Untersuchungsbereich befindlichen Teils des Untersuchungsobjekts moduliert und mit dieser modulierten Frequenz und/oder Phase wieder abgestrahlt wird. Mit einer Empfangsspulenanordnung, die Teil der MR-Anordnung ist, wird als MR-Signal sowohl das von der Spulenanordnung gesendete (modulierte) Spulensignal als auch ein Objektsignal aus dem angeregten Bereich des Untersuchungsobjekts empfangen. Da diese Signale unterschiedliche Frequenzen und/oder Phasen aufweisen, ist auf einfache Weise eine Trennung des Spulensignals und des Objektsignals möglich, so daß auch die Bestimmung der Position der Spulenanordnung und damit der Position des medizinischen Instruments einfach möglich ist.

Auch die in Verbindung mit einer Hochfrequenzleitung beschriebenen Nachteile treten demnach bei der erfindungsgemäßen MR-Anordnung nicht mehr auf, da weder von der Spulenanordnung an die Empfängeranordnung noch in umgekehrter Richtung Hochfrequenzsignale verlustarm übertragen werden müssen. Die bei der bekannten MR-Anordung erforderlichen niederohmigen Hochfrequenzleitungen können deshalb durch extrem dünne und hochohmige Leitungen ersetzt werden. Dadurch entfällt auch die Gefahr von Gewebeaufheizungen bei der erfindungsgemäßen MR-Anordnung und es ist kein zusätzlicher Empfangskanal oder eine Umschaltvorrichtung erforderlich.

Bei der bevorzugten Ausgestaltung gemäß Anspruch 2 sind die Elemente des Resonanzkreises und die Modulationseinheit benachbart zueinander, beispielsweise im Abstand von einigen Millimetern bis Zentimetern voneinander angeordnet, was den Vorteil hat, daß keine oder nur sehr kurze Hochfrequenzleitungen zwischen diesen Elementen erforderlich sind.

Die Steuerleitung bei der Weiterbildung der Erfindung gemäß Anspruch 3 ist bevorzugt als hochohmige Zweidrahtleitung ausgestaltet, deren Länge beliebig gewählt sein kann und über die der Modulationseinheit ein niederfrequentes Steuersignal von der Steuereinheit zugeführt wird. Die Steuereinheit ist bevorzugt außerhalb des Untersuchungsobjekts angeordnet.

Eine besonders einfache Möglichkeit der Steuerleitung bietet die Ausgestaltung der Erfindung gemäß Anspruch 4, bei der das Steuersignal auf optischem Wege zugeführt und mit geeigneten Mitteln, z.B. mit einer Art Optokoppler in ein elektrisches Steuersignal gewandelt wird.

Die Modulationseinheit kann besonders einfach und platzsparend aufgebaut sein und weist im wesentlichen gemäß Anspruch 5 eine Schalteranordnung auf, die in der entsprechenden geringen Größe hergestellt werden kann. Bevorzugt wird die Schalteranordnung zwischen zwei Schaltzuständen umgeschaltet zur Modulation der Schwingung des Resonanzkreises.

Die bevorzugte Weiterbildung gemäß Anspruch 6 stellt eine völlig drahtlose Lösung dar, bei der das Steuersignal als Hochfrequenz-Steuersignal in eine Hochfrequenz-Empfangsanordnung induziert und in das eigentliche Steuersignal für den Resonanzkreis umgewandelt wird. Die Frequenz des Hochfrequenz-Steuersignals ist dabei deutlich größer oder kleiner als die Frequenz des in die Spulenanordnung induzierten Signals und als die Resonanzfrequenz des Resonanzkreises, um Störungen zu vermeiden. Vorteilhafterweise ist auch die Hochfrequenz-Empfangsanordnung eng benachbart, beispielsweise im Abstand von einigen Zentimetern zu der Modulationseinheit und dem Resonanzkreis angeordnet.

Die Ausgestaltung gemäß Anspruch 8 ermöglicht insbesondere die Bestimmung der Lage des medizinischen Instruments in einem größeren Bereich, in dem die Spulen angeordnet sind, indem die Position der einzelnen Spulen bestimmt wird, deren Position in oder an dem Instrument bekannt ist.

Gemäß Anspruch 9 können auch mehrere Resonanzkreise vorgesehen sein, die auch unterschiedlich moduliert werden, z.B. mit unterschiedlichen Frequenzen, so daß die Position jeder einzelnen Spulenanordnung eindeutig bestimmbar ist, indem z.B. jede Spulenanordnung in einem anderen Bildbereich abgebildet wird.

Die Aufgabe betreffend das Verfahren zur Positionsbestimmung eines medizinischen Instruments wird dadurch gelöst, daß die Spulenanordnung mit einem Kondensator einen Resonanzkreis bildet, daß ein in die Spulenanordnung eingekoppeltes Hochfrequenzsignal derart moduliert wird, daß im MR-Signal ein moduliertes und von der Spulenanordnung wieder abgestrahltes Spulensignal enthalten ist, und daß aus dem Spulensignal die Position des Instruments ermittelt wird.

Bei dem erfindungsgemäßen Verfahren ist eine deutlich einfachere Positionsbestimmung der Spulenanordnung als bei dem bekannten Verfahren möglich. Durch die erfindungsgemäße Modulation, bei der dem in die Spulenanordnung induzierten Signal eine Frequenzverschiebung oder eine Phasendrehung aufgeprägt wird, ist das von der Spulenanordnung wieder abgestrahlte modulierte Spulensignal mit einfachen Rechenverfahren aus dem von der Empfangsspulenanordnung empfangenen MR-Signal abtrennbar.

In den Ansprüchen 12 bis 14 sind vorteilhafte Ausgestaltungen des erfindungsgemäßen Verfahrens angegeben, insbesondere vorteilhafte Arten der Modulation.

Die Erfindung wird nachfolgend anhand der Zeichnung näher erläutert. Es zeigen:
- Fig. 1: ein Blockschaltbild einer erfindungsgemäßen MR-Anordnung,
- Fig. 2: ein Ersatzschaltbild einer ersten Ausführungsform der Erfindung,
- Fig. 3: ein Ersatzschaltbild einer zweiten Ausführungsform der Erfindung,
- Fig. 4: ein Ersatzschaltbild einer dritten Ausführungsform der Erfindung mit drahtloser Zuführung des Steuersignals,
- Fig. 5: ein Ersatzschaltbild einer vierten Ausführungsform der Erfindung mit optischer Zuführung des Steuersignals,
- Fig. 6: ein Ersatzschaltbild einer Ausführungsform der Erfindung mit Mitteln zur Entdämfpung des Resonanzkreises,
- Fig. 7: eine Darstellung des k-Raumes zur Erläuterung des erfindungsgemäßen Verfahrens und
- Fig. 8: eine Darstellung gemessener Signale.

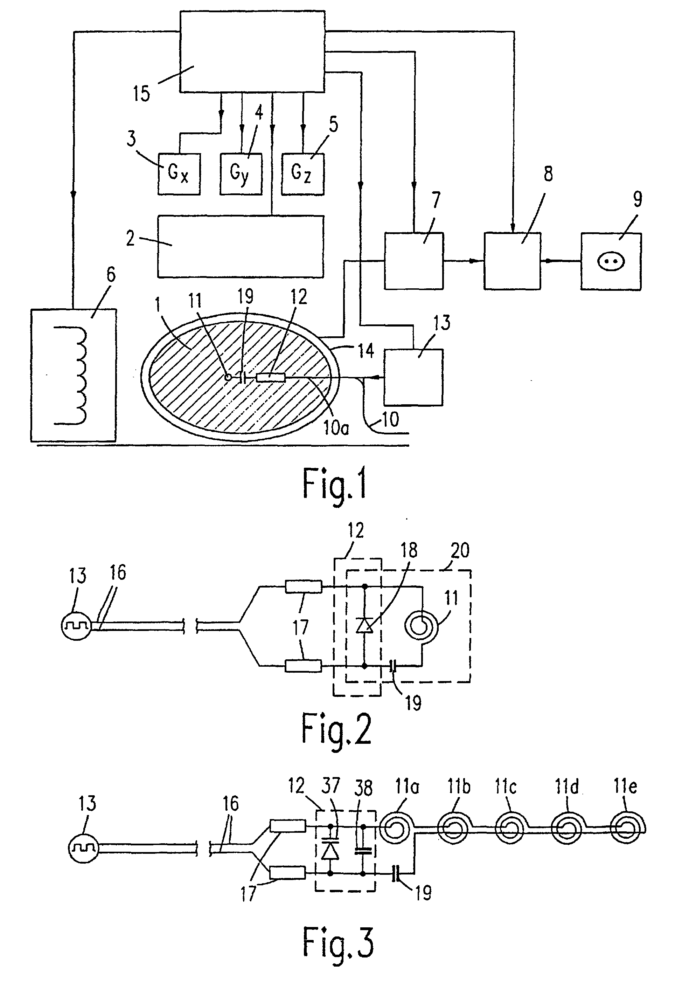

In Fig. 1 ist mit 1 ein Untersuchungsobjekt bezeichnet, das sich in einem Untersuchungsbereich befindet, der einem homogenen stationären Magnetfeld ausgesetzt ist, das von einem Haupfeldmagneten 2 erzeugt wird. Das stationäre homogene Magnetfeld kann mittels dreier Gradientenanordnungen 3, 4, 5 modifiziert werden, die ein magnetisches, in Richtung des homogenen stationären Magnetfeldes verlaufendes Gradientenfeld erzeugen, das in x-, y- oder z-Richtung einen Gradienten aufweist. Weiterhin ist ein Hochfrequenz-Sender 6 vorgesehen, der im Untersuchungsbereich impulsweise ein hochfrequentes Magnetfeld erzeugen kann.

Die im Untersuchungsobjekt erzeugten Objektsignale werden von einer Empfangsspulen-Anordnung 14, die aus einer oder mehreren Empfangsspulen bestehen kann, in Verbindung mit einer Empfängeranordnung 7 detektiert. Aus den digitalisierten Objektsignalen wird nach einer geeigneten Transformation, z.B. einer Fourier-Transformation in einer Rekonstruktionseinheit 8 die Kernmagnetisierungsverteilung im Untersuchungsbereich rekonstruiert und in Form eines MR-Bildes auf einer Wiedergabeeinheit 9 wiedergegeben.

In das Untersuchungsobjekt 1 ist ein medizinisches Instrument 10 eingeführt, beispielsweises ein Katheter, an dessen Spitze eine Mikrospule 11 befestigt ist, die einen in der US 5,353,795 beschriebenen Aufbau haben kann. In oder an dem in das Untersuchungsobjekt 1 eingeführten Teil 10a des Katheters 10 ist in der Nähe der Mikrospule 11 ein Kondensator 19, der mit der Mikrospule 11 einen Resonanzkreis bildet, und eine Modulationseinheit 12 angeordnet. Die Modulationseinheit 12 wird von einer Steuereinheit 13 gesteuert. Das in die Mikrospule 11 induzierte und nach einer Modulation durch die Modulationseinheit 12 von der Mikrospule 11 wieder abgestrahlte modulierte Spulensignal wird ebenfalls von der Empangsspulen-Anordnung 14 erfaßt (das Spulensignal der Mikrospule koppelt in die Empfangssspulen-Anordnung 14) und von den Komponenten 7, 8 verarbeitet. Die Komponenten 2 bis 8 sowie 13 werden von einer programmierbaren Steuereinheit 15 gesteuert.

In Fig. 2 ist eine erste Ausführungsform der Erfindung dargestellt. Die Mikrospule 11 bildet zusammen mit dem Kondensator 19 einen Resonanzkreis 20, dessen Resonanzfrequenz auf die Larmor-Frequenz des zu untersuchenden Gewebes des Untersuchungsobjekts 1 (z.B. auf die Larmor-Frequenz von Wasser) abgestimmt ist. In den Resonanzkreis 20 ist außerdem eine PIN-Diode 18 eingefügt, die die Modulationseinheit 12 bildet. Die PIN-Diode 18 ist über zwei Widerstände oder Drosselspulen 17 und eine hochohmige Zweidrahtleitung 16 mit einer Steuereinheit 13 verbunden, die eine rechteckförmige Wechselspannung liefert und damit die PIN-Diode 18 periodisch zwischen Sperr- und Durchgangszustand umschaltet. Dadurch wird erreicht, daß die Spule 11 abwechselnd periodisch ein Signal sendet (im Durchgangszustand der PIN-Diode 18) und kein Signal sendet (im Sperrzustand der PIN-Diode 18). Da die Schaltfrequenz deutlich niedriger ist als die Resonanzfrequenz des Resonanzkreises 20, wird folglich eine Modulation des in die Spule 11 induzierten Signals bewirkt, was sich im Frequenzbereich im wesentlichen als eine Verschiebung des Signals auswirkt. Die Modulationsfrequenz kann dabei so gewählt werden, daß das Spulensignal um einen bekannten Betrag im Frequenzbereich verschoben wird, so daß das Spulensignal vom Objektsignal getrennt wird. Dadurch wird erreicht, daß mit geeigneten rechnerischen Mitteln in der Empfängereinheit 7 oder der Rekonstruktionseinheit 8, z.B. durch eine Filterung im Zeit- oder Frequenzbereich, das Spulensignal identifiziert und daraus die Position der Spule 11 bestimmt und gegebenenfalls im MR-Bild dargestellt werden kann.

Die Empfangsspulen-Anordnung 14 ist in einem ausreichend großen Frequenzbereich abgestimmt, um neben den Objektsignalen auch die Spulensignale empfangen zu können. Während der Anregung durch den Hochfrequenz-Sender 6 kann die PIN-Diode 18 in den Sperrzustand versetzt werden, um lokale Veränderungen der Anregungsfeldstärke durch den Resonanzkreis 20 zu verhindern. Die PIN-Diode 18 kann jedoch auch während der Anregung im Durchgangszustand geschaltet sein, um eine höhere Signalamplitude von der Spule 11 zu erhalten.

Eine zweite Ausführungsform der Erfindung ist in Fig. 3 dargestellt. Mehrere Mikrospulen 11a bis 11e sind dabei in Serie geschaltet und mit dem Kondensator 19 auf die Larmor-Frequenz abgestimmt. Die Modulationseinheit 12 wird hier durch einen Varaktor (eine Kapazitätsdiode) 37 gebildet und ist wiederum über eine hochohmige Steuerleitung 16 mit der Steuereinheit 13 verbunden.

Durch die Verwendung mehrerer Mikrospulen 11a bis 11e, die in geringen Abständen von wenigen Millimetern oder Zentimetern entlang des Katheters 10 angeordnet sind, ist es möglich, den Verlauf des Katheters innerhalb des Untersuchungsobjekts 1 über eine größere Strecke zu bestimmen und im Bild darzustellen. Neben der Abstimmung auf die Larmor-Frequenz in Kombination mit dem Kondensator 38 und dem Varaktor 37 bewirkt der Kondensator 19 auch, daß der Varaktor 37 durch die Spulen 11a bis 11e gleichspannungsmäßig nicht kurzgeschlossen ist. Außerdem bewirkt der Kondensator 19 während der Sendephase zusammen mit dem Kondensator 38 eine Aufteilung der induzierten HF-Spannung derart, daß die reduzierte HF-Spannung am Varaktor 37 klein genug ist, diesen nicht zu verstimmen oder unerwünschte Signalkomponenten zu erzeugen. Außerdem kann die Abstimmempfindlichkeit der Resonanzfrequenz reduziert werden.

Ein Varaktor 37 hat gegenüber einer PIN-Diode 18 im wesentlichen den Vorteil, daß dessen Kapazität spannungsabhängig ist und daß deshalb auch bei Fertigungstoleranzen von Spulen und Kondensatoren der Resonanzkreis auf die genaue Larmor-Frequenz abgestimmt werden kann. In anderen Ausgestaltungen sind damit auch andere Modulationsarten als mit einer PIN-Diode möglich.

Bei der in Fig. 3 gezeigten Ausführungsform könnte anstelle des Varaktors auch eine PIN-Diode 18 vorgesehen sein. Es könnten auch nur eine Mikrospule 11a oder eine andere Anzahl von Mikrospulen 11a bis 11e vorgesehen sein. Es könnte auch der Kondensator 38 entfallen. Denkbar ist auch, den Kondensator 19 statt an der gezeigten Stelle zwischen den oberen Anschlüssen des Kondensators 38 und des Elements 37 anzuordnen.

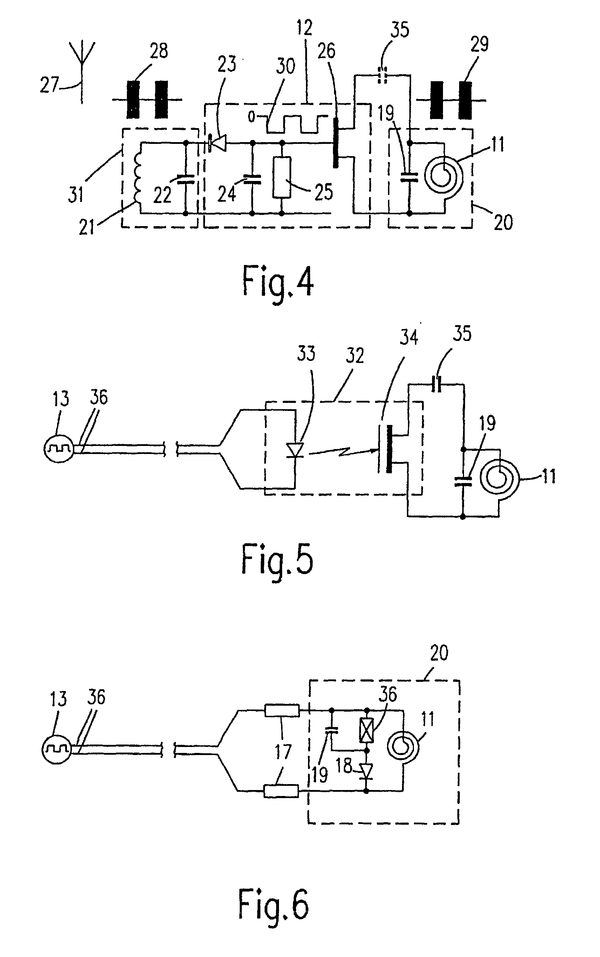

Eine Ausführungsform mit einer drahtlosen Steuerung der Modulationseinheit 12 ist in Fig. 4 gezeigt. Dort ist ein Empfänger-Resonanzkreis 31, der durch eine Parallelschaltung aus einer Spule 21 und einem Kondensator 22 gebildet ist, vorgesehen zum Empfang eines von einer Sendeeinheit 27 eingestrahlten Hochfrequenz-Steuersignals 28. Der Resonanzkreis 31 ist auf die Frequenz des Hochfrequenz-Steuersignals 28 abgestimmt, die in einem anderen Frequenzbereich liegt als die Larmor-Frequenz und die Resonanzfrequenz des Resonanzkreises 20, um gegenseitige Störungen zu vermeiden. Das Hochfrequenz-Steuersignal 28 weist, wie gezeigt, eine rechteckförmige Hüllkurve auf. Der Resonanzkreis 20 ist bei dieser Ausgestaltung als Parallelschaltung aus der Spule 11 und einem Kondensator 19 gebildet.

Die mit dem Resonanzkreis 31 verbundene Modulationseinheit 12 weist hier einen Feldeffekt-Transistor 26 auf, an dessen Gate G eine Gate-Spannung 30 anliegt, die den Transistor 26 periodisch in den niederohmigen bzw. hochohmigen Zustand steuert. Die Gate-Spannung 30 wird mittels einer mit dem Resonanzkreis 31 verbundenen Diode 23 und einer Parallelschaltung aus einem Kondensator 24 und einem Widerstand 25 aus dem von dem Resonanzkreis 31 empfangenen Hochfrequenz-Steuersignal 28 abgeleitet. Durch das periodische Umschalten des Transistors 26 wird dem Resonanzkreis 20 die gewünschte Modulation aufgeprägt, wobei die Modulationsfrequenz durch die Frequenz der rechteckförmigen Hüllkurve des Hochfrequenz-Steuersignals 28 einstellbar ist.

Anstelle eines Feldeffekt-Transistors 26 kann bei der gezeigten Ausführungsform auch ein anderes steuerbares Widerstands- oder Schaltelement eingesetzt werden, das periodisch mittels eines Steuerspannung in leitenden und nicht-leitenden Zustand oder hochohmigen und niederohmigen Zustand versetzt werden kann. Weiterhin kann zwischen dem Drain-Anschluß D des Transistors 26 und dem Resonanzkreis 20 ein (gestrichelt angedeuteter) Kondensator 35 vorgesehen sein, durch den die Spule 11 auf zwei unterschiedliche Resonanzfrequenzen abgestimmt werden kann: auf eine erste Resonanzfrequenz nur mit dem Kondensator 19 (im Sperrzustand des Transistors 26) und auf eine zweite Resonanzfrequenz mit der Parallelschaltung der Kondensatoren 19 und 35 (im leitenden Zustand des Transistors 26). Dadurch sendet die Spule 11 abwechselnd mit unterschiedlichen Phasen und ggf. auch mit unterschiedlichen Amplituden, wodurch wiederum das gesendete Spulensignal im empfangenen MR-Signal identifizierbar ist.

In einer bevorzugten Ausführungsform kann der Resonanzkreis 20 so abgestimmt werden, daß sich in beiden Abstimmzuständen gleiche Signal-Amplituden aber eine entgegengesetzte Phasendifferenz (bezüglich der Phase bei Abstimmung des Resonanzkreises 20 auf die Larmor-Frequenz) einstellt. Damit wird eine reine Phasenmodulation erreicht.

Eine Ausführungsform mit einer optischen Steuerleitung 36 von der Steuereinheit 13 an die Modulationseinheit 32 ist in Fig. 5 gezeigt. Die Steuerleitungen 36, die beispielsweise dünne Glasfaserkabel sein können, sind mit einer Leuchtdiode 33 verbunden. Diese erzeugt ein optisches Steuersignal, mit dem ein geeignetes, optisch steuerbares Schaltelement, beispielsweise ein optisch steuerbarer Transistor 34, gesteuert und dadurch periodisch in leitenden und nicht-leitenden Zustand versetzt wird. Die übrige Funktionsweise gleicht im wesentlichen der im Zusammenhang mit Fig. 4 beschriebenen Funktionsweise.

Als Modulationseinheit 32 ist auch der Einsatz eines einzigen Bauelements, z.B. eines geeigneten Optokopplers vorstellbar.

Neben den beschriebenen Ausführungsformen sind auch weitere Möglichkeiten der Ausgestaltung der Modulationseinheit denkbar. Beispielsweise könnte die Steuereinheit 13 völlig innerhalb des einführbaren Teils 10a (siehe Fig. 1) des medizinischen Instruments 10 angeordnet sein, z.B. als integrierter Rechteck-Modulator mit eingebauter Batterie. Es könnte auch eine Frequenz-Umsetzer-Schaltung mit Verstärkern in das medizinische Instrument 10 integriert werden. Darüber hinaus sind auch andere Schalteranordnungen mit anderen Elementen anstelle der gezeigten PIN-Diode, des Varaktors oder des Transistors denkbar.

Eine Ausführungsform mit Mitteln zur Erhöhung der Güte des Resonanzkreises 20 ist in Fig. 6 dargestellt. Parallel zum Kondensator 19 ist hier ein Element 36 mit negativem Widerstand (ein negativer Impedanzkonverter) geschaltet, das eine Entdämpfung des Resonanzkreises bewirkt und dadurch eine Erhöhung der Güte des Resonanzkreises 20 bewirkt. Dies hat den Vorteil, das das von der Spule 11 ein deutlich stärkeres Signal in die Empfangsspulenanordnung gekoppelt werden kann. Die Realisierung des Elements 36 kann unterschiedlich erfolgen, beispielsweise durch einen rückgekoppelten Feldeffekttransistor.

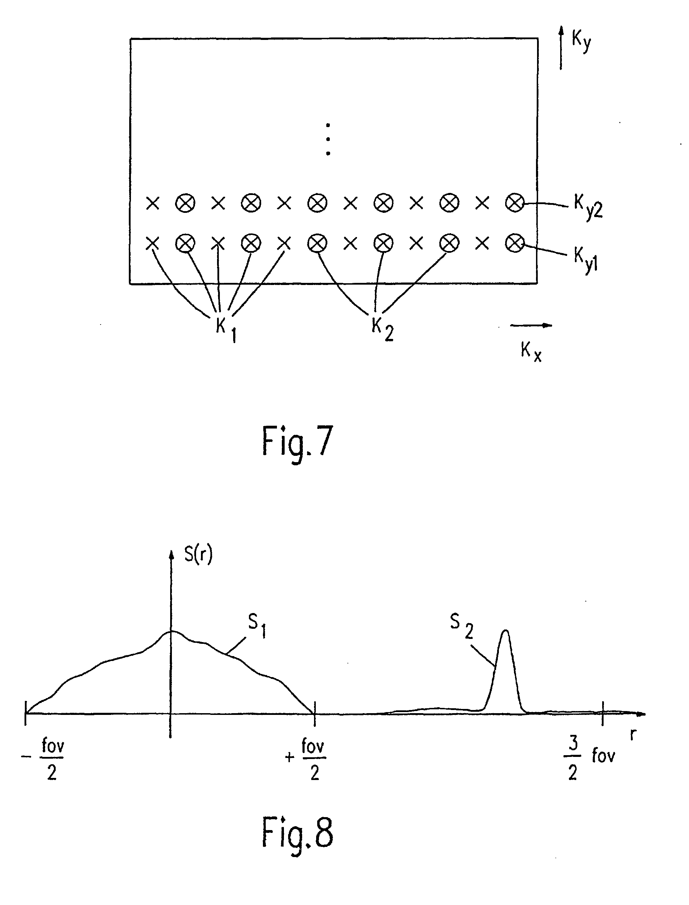

Anhand der Fig. 7 und 8 soll das erfindungsgemäße Verfahren näher erläutert und unterschiedliche Modulationsarten, die mit den in den Fig. 2 bis 6 gezeigten Ausführungsformen umgesetzt werden können, verdeutlicht werden.

Fig. 7 zeigt den k-Raum (Ortsfrequenzraum), der zur Erstellung eines MR-Bildes mit ausreichender Dichte abgetastet werden muß. Im gezeigten Beispiel wird der k-Raum entlang paralleler in kₓ-Richtung verlaufender Linien abgetastet. Die Abtastrate ist dabei so eingestellt, daß das von der Empfangsspulenanordnung gemessene MR-Signal, das das Objektsignal und das Spulensignal enthält, in gleichmäßigen Zeitabständen an den mit k₁ bezeichneten Stellen (x) abgetastet wird. Die Modulationsfrequenz wurde bei dem gezeigten Beispiel so gewählt, daß die Spule 11 nur zu jedem zweiten Abtastzeitpunkt, also nur an den mit k₂ (o) bezeichneten Stellen ein Signal liefert. Dies kann z.B. dadurch erreicht werden, daß das Steuersignal der Steuereinheit 13 duch Frequenzhalbierung aus der Abtastrate abgeleitet wird. Da von der Spule nur zu jedem zweiten Abtastpunkt k₂ ein Signal geliefert wird, ist dies gleichbedeutend mit einer Unterabtastung gegenüber dem Objektsignal, das zu jedem Abtastpunkt k₁ ein Signal liefert. Dies bewirkt eine Wiederholung der Abbildung der Spule im Ortsbereich an einer anderen Stelle. Bei der beschriebenen Wahl der Modulationsfrequenz ergibt sich eine Abbildung S₂ der Spule im Ortsbereich (r) horizontal versetzt um genau eine halbe Bildbreite. Wird die Abtastrate doppelt so groß gewählt (Überabtastung), wie zur Abbildung (des Field-of View = FOV) erforderlich ist, dann ergibt sich das resultierende MR-Bild doppelt so breit wie ein herkömmliches MR-Bild (Vergrößerung in Ausleserichtung), wobei das Objekt selbst zwischen -fov/2 und +fov/2 abgebildet wird (S₁) (siehe Fig. 8), während zwischen + 1/2 fov und 3/2 fov nur die Spule abgebildet ist. In dieser Bildhälfte läßt sich dann einfach die Spule identifizieren, ihre Position bestimmen und ggf. in die andere Bildhälfte einblenden.

Die Modulationsfrequenz kann auch derart gewählt werden, daß die Spule nicht zu jedem zweiten Abtastzeitpunkt (an den Stellen k₂ in Fig. 7), sondern nur entlang jeder zweiten horizontalen k-Linie ein Signal liefert, aber dann zu allen Abtastzeitpunkten k₁, z.B. daß die Spule nur entlang der Linien k_{y1}, k_{y3}, usw. nicht aber entlang der Linien k_{y2}, k_{y4}, usw. ein Signal liefert. Dadurch entsteht ein weiteres Abbild der Spule um genau eine halbe Bildhöhe vertikal versetzt (Vergrößerung des Bildes in Phasenkodierrichtung). Durch geeignete Wahl des FOV bzw. der Anzahl der gemessenen k-Linien kann erreicht werden, daß das zusätzliche Abbild der Spule in einem sonst "leeren" Bildbereich erscheint, wodurch wiederum eine einfach Identifikation und Lokalisation der Spule möglich ist.

Die Modulation könnte auch derart erfolgen, daß der Resonanzkreis derart gesteuert wird, daß die Spule abwechselnd auf die Larmorfrequenz und eine Frequenz ungleich der Larmorfrequenz abgestimmt ist. Denkbar ist auch eine Ansteuerung der Modulationseinheit mit einer sinusförmigen oder einer anderen Steuerspannung derart, daß dem Resonanzkreis eine Phasen- und/oder Frequenzmodulation aufgeprägt wird.

Eine Phasenmodulation kann beispielsweise dadurch erreicht werden, daß die Modulationseinheit den Resonanzkreis derart steuert, daß die Spule abwechselnd auf eine Frequenz oberhalb und unterhalb der Larmorfrequenz abgestimmt wird, wobei der Abstand der Resonanzfrequenzen zur Larmorfrequenz vorteilhafterweise etwa gleich groß gewählt wird, so daß sich in beiden Fällen eine etwa gleich große Signalamplitude ergibt. Zwar wird die Amplitude des gesendeten Signals dadurch etwas schwächer als bei Resonanz, ein Sendesignal tritt dafür aber in beiden Schaltzuständen auf. Die beiden Zustände unterscheiden sich dabei von der Phasenlage bei Resonanz um jeweils ±ϕ, z.B. um ±45°, so daß sich insgesamt ein Phasenunterschied von 2ϕ, z.B. von 90° ergibt. Dieser Phasenunterschied kann ausgenutzt werden, um durch Differenzbildung zweier gemessener MR-Signale mit unterschiedlichen Phasen das Spulensignal zu ermitteln und daraus die Position der Spule zu berechnen. Dieses Verfahren eignet sich auch zur Anwendung bei einem Projektions-Rekonstruktionsverfahren und bei radialer Abtastung des k-Raumes.

Die Erfindung ist nicht auf eine bestimmte Art der Abtastung des k-Raumes, auf die beschriebenen Modulationsarten und die gezeigten Ausführungsbeispiele beschränkt. Wesentlich ist, daß durch die Modulationseinheit die Schwingung des Resonanzkreises in irgendeiner Weise moduliert wird, so daß aus dem gemessenen MR-Signal das Spulensignal abtrennbar und daraus die Position der Spule bestimmbar ist.

Für die Erfindung ist auch nicht die Art und die Anzahl der für die Spulenanordnung(en) verwendeten Spulen wesentlich. Beispielsweise könnten statt der gezeigten Mikrospulen Spulen mit einem größeren Durchmesser oder Spulenanordungen mit drei Spulen mit zueinander senkrecht verlaufenden Spulenachsen verwendet werden, die nebeneinander oder um einen gemeinsamen Mittelpunkt angeordnet sind.

Es ist auch denkbar, mehrere Resonanzkreise mit je einer Spulenanordnung (z.B. mit je einer Mikrospule) parallel zu betreiben mit jeweils einer zugeordneten Modulationseinheit und die Resonanzkreise unterschiedlich (z.B. mit unterschiedlichen Frequenzen) zu modulieren, wodurch die Spulensignale der Spulenanordnungen in verschiedenen Bildbereichen des MR-Bildes erscheinen und deshalb die einzelnen Spulenanordnungen identifiziert und lokalisiert werden können.

## Patentansprüche

1. MR-Anordnung mit einem medizinischen Instrument (10) zur Einführung in ein Untersuchungsobjekt (1) und einer in oder an dem Instrument (10) angebrachten Spulenanordnung (11), die mit einem Kondensator (19) einen Resonanzkreis (20) bildet und die mindestens eine Spule umfaßt,
**dadurch gekennzeichnet, daß** an der Spulenanordnung (11) eine Modulationseinheit (12) angebracht ist zum Modulieren eines in die Spulenanordnung (11) eingekoppelten und von der Spulenanordnung wieder abgestrahlten Hochfrequenzsignals.

2. MR-Anordnung nach Anspruch 1,
**dadurch gekennzeichnet, daß** die Modulationseinheit (12), der Kondensator (19) und die Spulenanordnung (11) in geringem Abstand zueinander und in oder an dem in das Untersuchungobjekt (1) einführbaren Teil (10a) des Instruments (10) angeordnet sind.

3. MR-Anordnung nach Anspruch 1,
**dadurch gekennzeichnet, daß** zwischen einer Steuereinheit (13) zur Steuerung der Modulationseinheit (12) und der Modulationseinheit eine Steuerleitung (16) angeordnet ist.

4. MR-Anordnung nach Anspruch 3,
**dadurch gekennzeichnet, daß** die Steuerleitung (36) ein Glasfaserkabel ist und daß die Modulationseinheit (32) ausgestaltet ist zur Umwandlung eines optischen Steuersignals in ein elektrisches Steuersignal.

5. MR-Anordnung nach Anspruch 1,
**dadurch gekennzeichnet, daß** die Modulationseinheit (12) eine Schalteranordnung, insbesondere mit einer Diode (18), einem Varaktor (37), einem Transistor (26) oder einem integrierten Schaltelement aufweist.

6. MR-Anordnung nach Anspruch 1,
**dadurch gekennzeichnet, daß** eine Hochfrequenz-Empfangsanordnung (31), insbesondere ein Empfänger-Resonanzkreis mit einer Spule (21) und einem Kondensator (22) vorgesehen ist zum Empfang eines von einer Hochfrequenz-Steuereinheit (27) gesendeten Hochfrequenz-Steuersignals (28) und daß die Modulationseinheit (12) Mittel zur Umwandlung des Hochfrequenz-Steuersignals (28) in ein Modulationssignal (29) für den Resonanzkreis (20) aufweist.

7. MR-Anordnung nach Anspruch 6,
**dadurch gekennzeichnet, daß** die Hochfrequenz-Steuereinheit (27) und die Modulationseinheit (12) zur drahtlosen Übertragung des Hochfrequenz-Steuersignals (28) ausgestaltet sind.

8. MR-Anordnung nach Anspruch 1,
**dadurch gekennzeichnet, daß** die Spulenanordnung (11) mehrere in Reihe geschaltete, insbesondere in geringem Abstand zueinander angeordnete Spulen (11a, 11b, 11c, 11d, 11e) aufweist.

9. MR-Anordnung nach Anspruch 1,
**dadurch gekennzeichnet, daß** in oder an dem Instrument (10) mehrere Resonanzkreise (20) mit jeweils einer zugeordneten Modulationseinheit (12) angeordnet sind, wobei die Modulationseinheiten (12) die in die Spulenanordnung (11) eingekoppelten Hochfrequenzsignale unterschiedlich modulieren.

10. Medizinisches Instrument (10) zum Einführen in ein Untersuchungsobjekt (1), insbesondere Katheter oder Endoskop, mit einer in oder an dem Instrument (10) angebrachten Spulenanordnung (11) mit mindestens einer Spule zum Empfangen und/oder Senden eines Hochfrequenzsignals, wobei
die Spulenanordnung (11) mit einem Kondensator (19) einen Resonanzkreis (20) bildet,
**dadurch gekennzeichnet, daß** an der Spulenanordnung eine Modulationseinheit (12) angebracht ist zum Modulieren eines in die Spulenanordnung (11) eingekoppelten und von der Spulenanordnung wieder abgestrahlten Hochfrequenzsignals.

11. Verfahren zur Positionsbestimmung eines in ein Untersuchungsobjekt (1) einführbaren medizinischen Instruments (10), wobei das Untersuchungsobjekt (1) im Untersuchungsbereich einer MR-Anordnung angeordnet ist, wobei in oder an dem medizinischen Instrument (10) eine Spulenanordnung (11) angebracht ist, die mit einem Kondensator (19) einen Resonanzkreis (20) bildet und die mindestens eine Spule umfaßt, und wobei aus einem von einer Empfangsspulenanordnung (14) empfangenen MR-Signal die Position des Instruments (10) ermittelt wird,
**dadurch gekennzeichnet, daß** ein in die Spulenanordnung (11) eingekoppeltes Hochfrequenzsignal derart moduliert wird, daß im MR-Signal ein moduliertes und von der Spulenanordnung wieder abgestrahltes Spulensignal enthalten ist, und daß aus dem Spulensignal die Position des Instruments ermittelt wird.

12. Verfahren nach Anspruch 11,
**dadurch gekennzeichnet, daß** zur Modulation die Schwingung des Resonanzkreises (20) mit einer Schaltfrequenz an- und abgeschaltet wird.

13. Verfahren nach Anspruch 11,
**dadurch gekennzeichnet, daß** die Modulation derart erfolgt, daß der Resonanzkreis (20) abwechselnd auf eine Frequenz gleich bzw. ungleich der Frequenz des in die Spulenanordnung (11) eingekoppelten Hochfrequenzsignals abgestimmt wird.

14. Verfahren nach Anspruch 11,
**dadurch gekennzeichnet, daß** die Modulation derart erfolgt, daß der Resonanzkreis (20) abwechselnd auf eine Frequenz oberhalb bzw. ungleich der Frequenz des in die Spulenanordnung (11) eingekoppelten Hochfrequenzsignals abgestimmt wird.

## Claims

1. An MR device which is provided with a medical instrument (10) which is to be introduced into an object (1) to be examined, and also with a coil system (11) which is arranged in or on the instrument (10), constitutes a resonant circuit (20) in conjunction with a capacitor (19) and includes at least one coil, **characterized in that** the coil system (11) is provided with a modulation unit (12) for modulating an RF signal which is coupled into the coil system (11), and emitted again by the coil system.

2. An MR device as claimed in claim 1, **characterized in that** the modulation unit (12), the capacitor (19) and the coil system (11) are arranged at a small distance from one another and in or on the part (10a) of the instrument (10) that can be introduced into the object (1) to be examined.

3. An MR device as claimed in claim 1, **characterized in that** a control lead (16) is provided between a control unit (13) for controlling the modulation unit (12) and the modulation unit.

4. An MR device as claimed in claim 3, **characterized in that** the control lead (36) is an optical fiber cable and that the modulation unit (32) is arranged to convert an optical control signal into an electrical control signal.

5. An MR device as claimed in claim 1, **characterized in that** the modulation unit (12) includes a switch arrangement, which includes notably a diode (18), a varactor (37), a transistor (26) or an integrated switching element.

6. An MR device as claimed in claim 1, **characterized in that** there is provided an RF receiving device (31), notably a receiver resonant circuit with a coil (21) and a capacitor (22), for receiving an RF control signal (28) transmitted by an RF control unit (27), and that the modulation unit (12) includes means for converting the RF control signal (28) into a modulation signal (29) for the resonant circuit (20).

7. An MR device as claimed in claim 6, **characterized in that** the RF control unit (27) and the modulation unit (12) are arranged for wireless transmission of the RF control signal (28).

8. An MR device as claimed in claim 1, **characterized in that** the coil system (11) comprises a plurality of coils (1la, 11b, 11c, 11d, 11e) which are connected in series and are arranged notably at a small distance from one another.

9. An MR device as claimed in claim 1, **characterized in that** a plurality of resonant circuits (20), each of which includes an associated modulation unit (12), are arranged in or on the instrument (10), and that the RF signals coupled into the coil system (11) are modulated differently by the modulation units (12).

10. A medical instrument (10) which is to be introduced into an object (1) to be examined, notably a catheter or an endoscope, and includes a coil system (11) which is arranged in or on the instrument (10) and includes at least one coil for receiving and/or transmitting an RF signal, in which the coil system (11) constitutes a resonant circuit (20) in conjunction with a capacitor (19), **characterized in that** there is provided a modulation unit (12) for modulating an RF signal which is coupled into the coil system (11) and emitted again by coil system.

11. A method of determining the position of a medical instrument (10) which can be introduced into an object (1) to be examined, the object (1) to be examined being arranged in the examination zone of an MR device, a coil system (11) which constitutes a resonant circuit (20) in conjunction with a capacitor (19) and which includes at least one coil for receiving and/or transmitting an RF signal being arranged in or on the medical instrument (10) and the position of the instrument (10) being determined from an MR signal received by a receiving coil system (14), **characterized in that** the coil system (11) modulates an RF signal coupled into the coil system (11) in such a manner that a modulated coil signal which is emitted again by the coil system is contained in the MR signal, and that the position of the instrument is determined from the coil signal.

12. A method as claimed in claim 11, **characterized in that** the oscillation of the resonant circuit (20) is switched on and off at a switching frequency for the purpose of modulation.

13. A method as claimed in claim 11, **characterized in that** the modulation is performed in such a manner that the resonant circuit (20) is tuned alternately to a frequency which is equal to and to a frequency which is not equal to the frequency of the RF signal coupled into the coil system (11).

14. A method as claimed in claim 11, **characterized in that** the modulation is performed in such a manner that the resonant circuit (20) is tuned alternately to a frequency above or unequal to the frequency of the RF signal coupled into the coil system (11).

## Revendications

1. Appareil RM avec un instrument médical (10) à introduire dans un objet à examiner (1) et un montage de bobines (11), placé dans ou sur l'instrument (10), qui forme un circuit de résonance (20) avec le condensateur (19) et comprend au moins une bobine, **caractérisé en ce qu'**une unité de modulation (12) est placée sur le montage de bobines (11) pour moduler un signal à haute fréquence couplé dans le montage de bobines (11) et rediffusé par le montage de bobines.

2. Appareil RM selon la revendication 1, **caractérisé en ce que** l'unité de modulation (12), le condensateur (19) et le montage de bobines (11) sont disposés à une distance minime l'un de l'autre et dans ou sur la partie (10a) de l'instrument (10) à introduire dans ou sur l'objet à examiner (1).

3. Appareil RM selon la revendication 1, **caractérisé en ce qu'**une ligne de commande (16) est disposée entre une unité de commande (13) pour la commande de l'unité de modulation (12) et l'unité de modulation.

4. Appareil RM selon la revendication 3, **caractérisé en ce que** la ligne de commande (36) est un câble en fibres de verre et que l'unité de modulation (32) est conçue pour la transformation d'un signal de commande optique en un signal de commande électrique.

5. Appareil RM selon la revendication 1, **caractérisé en ce que** l'unité de modulation (12) présente un circuit, en particulier une diode (18), une diode varicap (37), un transistor (26) ou un dispositif de commutation intégré.

6. Appareil RM selon la revendication 1, **caractérisé en ce qu'**un récepteur à haute fréquence (31), en particulier un circuit de résonance de récepteur avec une bobine (21) et un condensateur (22) est prévu pour la réception d'un signal de commande à haute fréquence (28) transmis par une unité de commande à haute fréquence (27) et que l'unité de modulation (12) présente des moyens pour la transformation du signal de commande à haute fréquence (28) en un signal de modulation (29) pour le circuit de résonance (20).

7. Appareil RM selon la revendication 6, **caractérisé en ce que** l'unité de commande à haute fréquence (27) et l'unité de modulation (12) sont conçues pour la transmission sans fil du signal de commande à haute fréquence (28).

8. Appareil RM selon la revendication 1, **caractérisé en ce que** le montage de bobines (11) présente plusieurs bobines (11a, 11b, 11c, 11d, 11e) montées en série disposées, en particulier à un faible intervalle l'une de l'autre.

9. Appareil RM selon la revendication 1, **caractérisé en ce que** plusieurs circuits de résonance (20) avec une unité de modulation (12) affectée respective sont disposés dans ou sur l'instrument (10), les unités de modulation (12) modulant différemment les signaux à haute fréquence couplés dans le montage de bobines (11).

10. Instrument médical (10) à introduire dans un objet à examiner (1), en particulier un cathéter ou un endoscope, avec un montage de bobines (11) placé dans ou sur l'instrument (10) avec au moins une bobine pour la réception et/ou la transmission d'un signal à haute fréquence, le montage de bobines (11) formant un circuit de résonance (20) avec un condensateur (19), **caractérisé en ce qu'**une unité de modulation (12) est placée sur le montage de bobines (11) pour la modulation d'un signal à haute fréquence couplé dans le montage de bobines (11) et rediffusé par le montage de bobines.

11. Procédé pour déterminer la position d'un instrument médical (10) à introduire dans un objet à examiner (1), l'objet à examiner (1) étant disposé dans la zone d'examen d'un appareil RM et un montage de bobines (11) qui forme un circuit de résonance (20) avec un condensateur (19) et comprend au moins une bobine étant placé dans ou sur l'instrument médical (10) et la position de l'instrument (10) étant déterminée à partir d'un signal RM reçu par un montage de bobines réceptrices (14), **caractérisé en ce qu'**un signal à haute fréquence couplé dans le montage de bobines (11) est modulé de telle sorte qu'un signal de bobines modulé et rediffusé par le montage de bobines est contenu dans le signal RM et que la position de l'instrument est déterminée à partir du signal de bobines.

12. Procédé selon la revendication 11, **caractérisé en ce que**, pour la modulation, l'oscillation du circuit de résonance (20) est activée ou arrêtée avec une fréquence de commutation.

13. Procédé selon la revendication 11, **caractérisé en ce que** la modulation intervient de telle sorte que le circuit de résonance (20) est syntonisé en alternance à une fréquence égale ou différente de la fréquence du signal à haute fréquence couplé dans le montage de bobines (11).

14. Procédé selon la revendication 11, **caractérisé en ce que** la modulation intervient de telle sorte que le circuit de résonance (20) est syntonisé en alternance à une fréquence supérieure ou différente de la fréquence du signal à haute fréquence couplé dans le montage de bobines (11).
